# EUROPEAN PATENT APPLICATION

(11) **EP 3 502 262 A1**
(43) Date of publication of application: **26.06.2019**
(21) Application number: 17306909.7
(22) Date of filing: 22.12.2017
(51) Int. Cl.: C12P 7/22, C12P 7/24, C12P 7/42

(54) **PROCESS FOR THE HYDROXYLATION OF PHENOLIC DERIVATIVES CATALYZED BY HPAB MONOOXYGENASE**

(71) Applicant: RHODIA OPERATIONS, 75009 Paris (FR); College de France, 75231 Paris Cedex 05 (FR)
(72) Inventor: Deng, Yifan, 75005 Paris (FR); Fontecave, Marc, 38330 Saint Ismier (FR); Back, Olivier, 69008 Lyon (FR)
(74) Representative: Delenne, Marc

(57) **Abstract**

The invention relates to an in vitro catalytic process for the regioselective aromatic hydroxylation of a hydroxyphenyl compound of formula (I) 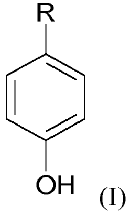 wherein R is selected from the group consisting of C₃-C₆ hydroxyalkyl groups, C₁-C₆ hydroxycarboxyalkyl groups and alkoxycarbonyl groups of formula - COOR¹, wherein R¹ is a C₁-C₆ alkyl group, said process comprising contacting in an aerobic aqueous and cell-free reaction medium said hydroxyphenyl compound with a HpaB monooxygenase in presence of (i) a flavin compound selected from FAD, FMN and mixtures thereof, (ii) a HpaC or Fre flavin reductase and (iii) a reducing agent being selected from a NADH compound or a NADPH compound.

SEQUENCE LISTING

<110>  RHODIA OPERATION
       COLLEGE DE FRANCE

<120>  PROCESS FOR THE HYDROXYLATION OF PHENOLIC DERIVATIVES CATALYZED
       BY HPAB MONOOXYGENASE

<130>  R 2017/110

<140>  EP 17306909.7
<141>  2017-12-22

<160>  2

<170>  PatentIn version 3.5

<210>  1
<211>  32
<212>  DNA
<213>  Escherichia coli

<220>
<221>  gene
<222>  (1)..(32)

<400>  1
cccgcatatg aaaccagaag atttccgcgc ca                                    32

<210>  2
<211>  34
<212>  DNA
<213>  Escherichia coli

<400>  2
tttttctcga gttatttcag cagcttatcc agca                                  34

## Description

### TECHNICAL FIELD

The present invention relates to a process for the hydroxylation of phenolic derivatives using HpaB monooxygenase as a catalyst under aerobic in vitro conditions.

### BACKGROUND ART

Di- and tri-hydroxyphenyl compounds are valuable chemical compounds that can be used as chemical intermediates for the synthesis of higher value products, such as vanillin for example. Those di- and tri-hydroxyphenyl compounds have been shown also to exhibit interesting properties, such as antioxidant properties. They may be used in different applications, for example in the pharmaceutical industry and in the fields of agronomics and cosmetics, or also as polymerization inhibitors.

The hydroxylation reaction is highly energy-consuming and difficult to carry out. Other disadvantages of the chemical route are the low regioselectivity and the low yields due to the formation of many by-products requiring therefore more complex and costly industrial manufacturing processes.

Enzymatic routes for hydroxylation of aromatic compounds have been developed in the past few years. At least five types of enzymes are able to catalyze aromatic hydroxylation: cytochrome P450-dependent monooxygenases, iron hydroxylases, copper monooxygenases, pterin monooxygenases and flavin monooxygenases. The first four classes of enzymes contain a metal center that is essential for their activity, whereas flavin monooxygenases do not contain metal, but an organic cofactor (flavin in the form of FAD or FMN). Furthermore, it has been shown that flavin monooxygenases are capable in vivo of hydroxylating compounds other than their natural substrates (such as the hydroxylation of coumaric acid to caffeic acid as reported in Appl. Microbiol. Biotechnol. 2014, 98, p. 1145).

Document US 2017/0037437 describes the hydroxylation of resveratrol to piceatannol under in vitro conditions using 4-hydroxyphenyl 3-hydroxylase.

The present invention aims at providing a process for the hydroxylation of phenolic derivatives catalyzed by a HpaB monooxygenase under aerobic in vitro conditions.

### SUMMARY OF THE INVENTION

A first object of the present invention relates to an in vitro catalytic process for the regioselective aromatic hydroxylation of a hydroxyphenyl compound of formula (I) 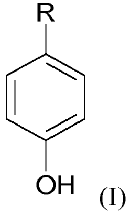 wherein R is selected from the group consisting of C₃-C₆ hydroxyalkyl groups, C₁-C₆ hydroxycarboxyalkyl groups and alkoxycarbonyl groups of formula - COOR¹, wherein R¹ is a C₁-C₆ alkyl group, said process comprising contacting in an aerobic aqueous and cell-free reaction medium said hydroxyphenyl compound with a HpaB monooxygenase in presence of (i) a flavin compound selected from FAD, FMN and mixtures thereof, (ii) a HpaC or Fre flavin reductase and (iii) a reducing agent being selected from a NADH compound or a NADPH compound.

Preferably, the hydroxyphenyl compound is selected from methyl-4-hydroxybenzoate and 4-hydroxymandelic acid.

According to an embodiment of the invention, the molar ratio of compound of formula (I) to HpaB monooxygenase ranges from 1000 to 10000.

According to an embodiment of the invention, the molar ratio of compound of formula (I) to the flavin compound ranges from 10 to 1000, preferably from 50 to 500.

According to an embodiment of the invention, the molar ratio of compound of formula (I) to the reducing agent ranges from 0.1 to 1.

As already mentioned, the process according to the invention is highly regioselective and tolerant to the presence of other chemical functionalities in said phenolic derivatives. Also, the process according to the invention relies on the use of O₂ (or air) as the oxidant and generates only H₂O as the by-product of the hydroxylation reaction.

Moreover, the process according to the invention allows satisfactory conversion of the hydroxyphenyl compound while keeping excellent selectivity.

### BRIEF DESCRIPTION OF THE DRAWINGS

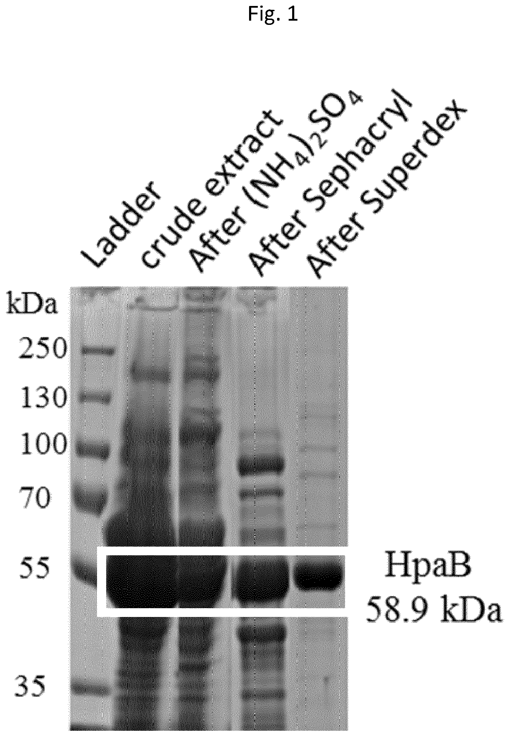

Figure 1 shows a denaturing SDS gel during purification of HpaB monooxygenase.

### DETAILED DESCRIPTION OF THE INVENTION

The present invention is directed to an in vitro catalytic process for the regioselective aerobic aromatic hydroxylation of a hydroxyphenyl compound of formula (I) 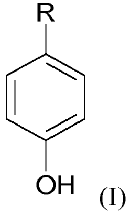 wherein R is selected from the group consisting of C₃-C₆ hydroxyalkyl groups, C₁-C₆ hydroxycarboxyalkyl groups and alkoxycarbonyl group of formula -COOR¹, wherein R¹ is a C₁-C₆ alkyl group, said process comprising contacting in an aerobic aqueous and cell-free reaction medium said hydroxyphenyl compound with a HpaB monooxygenase in presence of (i) a flavin compound selected from FAD, FMN and mixtures thereof, (ii) a HpaC or Fre flavin reductase and (iii) a reducing agent being selected from a NADH compound or a NADPH compound.

In a preferred embodiment of the invention, the compound of formula (I) is selected from methyl-4-hydroxybenzoate and 4-hydroxymandelic acid.

It has been surprisingly found that the process according to the invention is highly regioselective. Indeed, the hydroxylation of compound of formula (I) mainly occurs at the ortho position of the hydroxyl group, leading to the formation of the dihydroxyphenyl compound of formula (II) 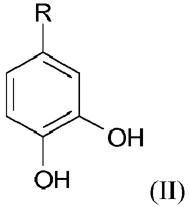 wherein R is as defined above.

In an embodiment of the invention, the hydroxylation of compound of formula (I) may also occur in both ortho positions of the hydroxyl group of compound of formula (I), resulting in the formation of the trihydroxyphenyl compound of formula (III) 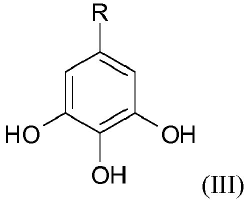 wherein R is as defined above.

According to an embodiment of the invention, the molar ratio of compound of formula (II) to compound of formula (III) is of at least 50/50, preferably at least 70/30, more preferably at least 80/20.

According to an embodiment of the invention, the initial conversion rate of compound of formula (I) is of at least 80 nmol/nmol HpaB/min, preferably at least 90 nmol/nmol HpaB/min.

The HpaB monooxygenase component used in the process according to the invention may be a subunit of a two-component flavin-dependent monooxygenase.

In an embodiment of the invention, the two-component flavin-dependent monooxygenase consists of a HpaB monooxygenase component and a flavin reductase (HpaC or Fre). This system is described for example in US 2017/0037432.

The flavin component used in the process according to the invention is selected from FAD, FMN and mixtures thereof. Preferably, the flavin component may be provided by a flavin reductase used in a FAD-containing form or in a FMN-containing form.

The source of HpaB monooxygenase and flavin reductase is not specifically limited and examples include bacteria classified into genera such as *Pseudomonas, Escherichia, Enterobacter, Krebsiella, Salmonella, Serratia, Shigella, Yersinia, Rhizobium, Bradyrhizobium, Mesorhizobium, Sinorhizobium, Bacillus, Geobacillus, Saccharothrix, Streptomyces, Rhodococcus, Gordonia, Mycobacterium, Thermus, Acinetbacter, Halomonas,* and *Micrococcus. Escherichia coli* is particularly preferable. It should be noted that HpaB and HpaC herein include all homologs and mutants which are capable of functioning as a two-component flavin-dependent oxygenase when interacted with each other, unless the enzyme is specified based on a species. Each homolog or mutant may have a natural sequence or an artificial sequence. HpaB and HpaC may be derived from the same species or different species.
The type of HpaB and HpaC is not specifically limited. They may independently be a recombinant protein or a natural protein.

According to the invention, a reducing agent is added to the reaction medium to reduce the flavin component in order to achieve the catalytic cycle. The reducing agent may be selected from a NADH compound or a NADPH compound.

The expression and purification methods of HpaB monooxygenase are described hereinafter in the examples.

The molar ratio of compound of formula (I) to HpaB monooxygenase ranges from 1000 to 10000.

The molar ratio of compound of formula (I) to the flavin compound ranges from 10 to 1000, preferably from 50 to 500.

The molar ratio of compound of formula (I) to the reducing agent ranges from 0.1 to 1.

The process according to the invention is performed in an aqueous medium. In an embodiment of the invention, the pH of the reaction medium ranges from 5 to 9, preferably from 6 to 8.

The process according to the invention may be carried out at a temperature ranging from about 20 to about 45 °C, more preferably from 20 to 35 °C, for a time sufficient to convert the compound of formula (I) into the desired product, such as about 5 to 120 minutes, preferably about 10 minutes to 90 minutes, more preferably about 20 minutes to 60 minutes.

The process according to the invention may be performed as a batch process.

The present invention will now be illustrated by the following examples, which are not intended to be limiting.

### EXAMPLES

### 1. Enzyme expression and purification

### 1.a Plasmid construction

The gene *HpaB* used to construct the expression plasmid comes from the genomic DNA of *E.coli.* The extraction of DNA from *E.coli* BL21 is done according to the protocol QIAGEN Genomic DNA Handbook. To amplify the gene *HpaB,* a PCR was run by using the following primers:
HpaB F (Forward) CCCGCATATGAAACCAGAAGATTTCCGCGCCA
HpaB_R (Reverse) TTTTTCTCGAGTTATTTCAGCAGCTTATCCAGCA

The product of PCR is purified by agarose gel electrophoresis. The gene *HpaB* was cloned between two restriction sites: Nde I and Xho I of plasmid pET-22b. Before introducing the gene into the cloning site, the plasmid and the purified PCR product are digested by two restriction enzymes, Nde I and Xho I. The ligation between plasmid and amplified gene *HpaB* is done by following a standard protocol. Then, the product is introduced into *E.coli* DH5α to amplify the plasmid which contains the gene *HpaB.* The amplified plasmid is collected using the protocol Miniprep.

### 1.b Expression of HpaB

*E.coli* BL21 is transformed with the plasmid co-expressed with chaperones GroEL/GroES and grown at 37 °C (induction of 0.2% (w/v) L-arabinose at DO₆₀₀ₙₘ = 0.45). Induction with 100 µM IPTG is done when DO₆₀₀ₙₘ = 1.5 and then growth continues at 16 °C overnight. For large scale production of HpaB, we use 4 liters LB culture following the best conditions of expression. At the end, the bacteria are collected by centrifugation at 6000 rpm, 4 °C for 10 min.

### 1.c Purification of HpaB

The collected bacteria are suspended in a lyse buffer (50 mM Tris pH 7.5, 150 mM NaCl, 10% glycerol, 1 mM PMSF), the volume used is 5 times of the bacterial weight. The sonication (Branson Sonifier 450) takes 240 cycles with 5 s on - 15 s off at amplitude of 40%. These lysed cells are ultra-centrifuged at 40000 rpm, 4 °C for 1 hour.

The supernatant is treated by a fractional precipitation of (NH₄)₂SO₄ after the ultra-centrifugation. This step is done first by adding 40% (NH₄)₂SO₄ in the supernatant, then incubating at 4 °C for 30 min with shaking. After a centrifugation at 14000 rpm, 4 °C for 15 min, the supernatant is collected and 10% extra (NH₄)₂SO₄ is added in the solution (50% of (NH₄)₂SO₄ in total). After repeating the incubation and centrifugation, the precipitant is collected.

The precipitant is suspended in a buffer of 25 mM Tris pH 7.5, 50 mM NaCl, 10% glycerol, which is the mobile phase of the column Hiprep 26/60 sephacryl S-200HR. The purification on the column first equilibrated with the mobile phase is achieved in a FPLC system (ÄKTA Purifier 10). The samples collected after chromatography are analyzed by SDS-PAGE. All the fractions containing the protein are collected, mixed and concentrated with a 30 kDa membrane. The second column used is Hiload 26/600 Superdex 200pg which separates proteins according to their size, with 25 mM Tris pH 7.5, 150 mM NaCl as the mobile phase. The fractions are analyzed again by SDS-PAGE. Then the pure HpaB fractions were collected, combined and concentrated. 15% glycerol is added before freezing HpaB at -80 °C. The chromatogram of HpaB monooxygenase is illustrated in figure 1.

### 2. Enzymatic activity tests on different substrates

The enzymatic tests which convert 4-hydroxymandelic acid into its 3,4-dihydroxymandelic acid are carried out at room temperature in a total volume of 500 µL. It contains 1 mM of the substrate, 1 or 10 µM HpaB, 2 µM FAD, 0.5 µM Fre (a flavin reductase) and 2 mM NADH. The reaction is initiated by adding the enzyme in the solution and quenched by adding 1 % (v/v) of 100% TCA at different times. The samples are centrifuged at 13000 rpm for 5 min. Before injection into HPLC the pH of the sample is adjusted to around 4 and then 50 µL is injected by an auto-sampler. An Agilent 1200 Infinity HPLC system is used to analyze the samples. The column is Agilent Eclipse XDB-C18, 5 µm, 4.6×15 mm, the two mobile phases are A: 10 mM KH₂PO₄ pH 2.6 and B: acetonitrile, the flow rate is 1 mL/min. The typical gradient to analyze tyrosol and hydroxytyrosol is 5-15% B within 15 min and then pass down to 5% B for another 5 min. The gradient is modified according to the hydrophobicity of the product. As a Photodiode array is used as a detector, different wavelengths can be measured at the same time. The typical wavelength is 225 nm. Others like 250 and 280 nm are used depending on the product. The conversion from pick area to quantify is based on calibration curve which is established by analyzing standard samples. The final conversion rate is of 20%.

The same procedure has been followed to convert methyl 4-hydroxybenzoate to methyl 3,4-dihydroxybenzoate. The final conversion rate is of 10%.

The process according to the invention has also been carried out with the following substrates: catechol, dihydroxybenzoic acid, para-tertbutylphenol and 4-hydroxy-3-methoxyphenylacetic acid. No conversion of these substrates to the desired dihydroxyphenyl products was observed, which shows that the process according to the invention is only effective towards the compounds of formula (I) according to the invention.

## Claims

1. An in vitro catalytic process for the regioselective aromatic hydroxylation of a hydroxyphenyl compound of formula (I) 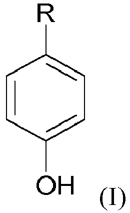 wherein R is selected from the group consisting of C₃-C₆ hydroxyalkyl groups, C₁-C₆ hydroxycarboxyalkyl groups and alkoxycarbonyl groups of formula -COOR¹, wherein R¹ is a C₁-C₆ alkyl group, said process comprising contacting in an aerobic aqueous and cell-free reaction medium said hydroxyphenyl compound with a HpaB monooxygenase in presence of (i) a flavin compound selected from FAD, FMN and mixtures thereof, (ii) a HpaC or Fre flavin reductase and (iii) a reducing agent being selected from a NADH compound or a NADPH compound.

2. The process according to claim 1, wherein R is a C₃-C₆ hydroxyalkyl group.

3. The process according to claim 1, wherein R is a C₁-C₆ hydroxycarboxyalkyl group.

4. The process according to claim 1, wherein R is an alkoxycarbonyl group of formula -COOR¹, wherein R¹ is a C₁-C₆ alkyl group.

5. The process according to claim 1, wherein the hydroxyphenyl compound is methyl-4-hydroxybenzoate.

6. The process according to claim 1, wherein the hydroxyphenyl compound is 4-hydroxymandelic acid.

7. The process according to any one of the preceding claims, wherein the flavin compound is FAD.

8. The process according to any one of the preceding claims but 7, wherein the flavin compound is FMN.

9. The process according to any one of the preceding claims, wherein a HpaC reductase is used as compound (ii).

10. The process according to any one of the preceding claims but 9, wherein a Fre flavin reductase is used as compound (ii).

11. The process according to any one of the preceding claims, wherein the reducing agent is a NADH compound.

12. The process according to any one of the preceding claims but 11, wherein the reducing agent is a NADPH compound.

13. The process according to any one of the preceding claims, wherein the molar ratio of compound of formula (I) to HpaB monooxygenase ranges from 1000 to 10000.

14. The process according to any one of the preceding claims, wherein the molar ratio of compound of formula (I) to the flavin compound ranges from 10 to 1000, preferably from 50 to 500.

15. The process according to any one of the preceding claims, wherein the molar ratio of compound of formula (I) to the reducing agent ranges from 0.1 to 1.
